# EUROPEAN PATENT APPLICATION

(11) **EP 4 354 453 A1**
(43) Date of publication of application: **17.04.2024**
(21) Application number: 22201290.8
(22) Date of filing: 13.10.2022
(51) Int. Cl.: G16H 30/40, H04L 65/75, H04N 21/8547

(54) **COMMUNICATING MEDICAL IMAGE DATA**

(30) Priority: 11.10.2022 US 202263415048 P
(71) Applicant: Koninklijke Philips N.V., 5656 AG Eindhoven (NL)
(72) Inventor: DE LANGE, Alphonsus Anthonius Jozef, Eindhoven (NL)
(74) Representative: Philips Intellectual Property & Standards

(57) **Abstract**

Proposed concepts aim to provide schemes, solutions, concepts, designs, methods and systems pertaining to the transmitting, receiving and/or communicating medical imaging data for remote real-time examination. In particular, a medical image is encoded (120) with an identifier based on at least part of metadata associated with the medical image. As the medical image and the metadata may have different transmission requirements (i.e. bandwidth, latency, loss, etc.), they are communicated through different communication channels (130, 140). In this way, the medical image and the metadata may be communicated appropriately, while the identifier enables the medical image and the metadata to be matched at a receiver.

## Description

### FIELD OF THE INVENTION

The present invention relates to communicating medical image data, and more particularly to the communication of medical imaging data for remote real-time examination.

### BACKGROUND OF THE INVENTION

Recently the process of transferring medical images (i.e., ultrasound images, magnetic resonance images, computed tomography images, etc.) in real-time to remote observers has been a subject of particular interest. There are many situations in which a dedicated trained medical professional is not able to be physically present at a scanning session. Specialized consultants may not be in the same medical facility, for example, or may need to be accessible for aiding many scans at once. Thus, providing concepts for enabling remote medical observation may enable improved patient outcomes.

However, a reduced latency of communication of the medical images must be achieved in order to avoid frustration and improve effectiveness of the scanning session. Medical imaging data can be particularly large, and therefore a communication link with a large bandwidth is essential to avoid excessive latency. This problem is compounded as remote real-time medical examination/observation typically requires an additional video communication channel between the remote observer(s) and the sonographer (i.e., the person physically performing the scan) for appropriate guidance and to ensure desired information is gathered.

Moreover, for remote observation there is usually a focus on the transmission of the ultrasound images, with no/little transfer of metadata (e.g., a gain, a depth, an acquisition mode, and a color map) related to the images to the remote observer. In contrast, many scanners/medical devices are able to export a medical image data stream in real-time. This data stream may not only contain image frames of a variety of different types, it may also contain metadata about the image frames. Such metadata may prove particularly useful for extended auditing purposes, as well as processing applications and/or services for feature recognition and quantification of the medical images.

### SUMMARY OF THE INVENTION

The invention is defined by the independent claims. Dependent claims define advantageous embodiments.

Proposed are concepts pertaining to communicating medical imaging data for remote real-time medical examination. In particular, a medical image is encoded with an identifier based on at least part of the metadata associated with the medical image. As the medical image and the metadata may have different transmission requirements (i.e., bandwidth, latency, loss, etc.), they are communicated through different communication channels. In this way, the medical image and the metadata may be communicated appropriately, while the identifier enables the medical image and the metadata to be matched at a receiver.

According to examples in accordance with an aspect of the invention, there is provided a method of transmitting medical imaging data for remote real-time examination, comprising:
obtaining a medical image and metadata comprising information describing one or more parameters of the medical image;
encoding the medical image with an identifier, the identifier based on at least part of the metadata;
transmitting the encoded medical image to a receiver by a first communication channel; and
transmitting the metadata to the receiver by a second communication channel different from the first communication channel.

As a result of transmitting the medical image and the associated metadata through different communication channels, the different parts of the medical imaging data may be more appropriately transmitted. Indeed, in some cases there may be provided a stream of medical image at the receiver by a fast data channel, whilst also benefitting from metadata received through a reliable data channel. This, and other benefits, are provided by the ability to send the different parts of the medical imaging data through different channels, whilst linking the medical image and the metadata via an identifier.

Put another way, medical imaging data may be received in real-time from a scanning device. The medical imaging data comprises a medical image/frame (or images/frames) of a subject, alongside metadata describing one or more parameters/properties of the image. It has been realized that the medical image and the metadata can be linked by using an identifier based on (at least part) of the metadata itself, which is encoded/embedded/linked into/to the medical image. Accordingly, this enables the separate transmission of the medical image and the metadata through different communication channels, whilst ensuring that the medical image and metadata remain linked (i.e., they can be matched at a receiver).

Thus, the invention enables the transmission of a medical image and associated metadata over different communication channels without introducing problems related to separately receiving images and metadata at the receiver.

As a result of the invention, real-time (i.e., as the medical image(s) are being acquired) remote (i.e., at a location separate from where the scanning is being conducted) examination may be improved. More specifically, the medical image(s) can be sent through a communication channel adapted for effective real-time transmission of the medical images, while the metadata may be sent more sparsely (but reliably) through a different communication channel.

Furthermore, the first communication channel and second communication channel may be selected based on their required characteristics. For example, the first communication channel may be more suited for transmitting a stream of medical images (i.e., have an appropriate bandwidth, latency characteristics, and security requirements). The second communication channel may be more suited for transmitting infrequently updated metadata (i.e., ensure accuracy/quality). Thus, system resources may be allocated more appropriately given transmission through different communication channels.

In some embodiments, encoding the medical image may comprise generating a machine-readable code based on the identifier, and embedding the machine-readable code within the medical image.

By embedding the medical image with a machine-readable code, the medical image and metadata may be automatically linked/matched at a receiver by a machine. This may enable efficient and quick linking of the medical image and metadata, meaning that the transmission of the medical image and the metadata through different communication channels may not be known to the user (i.e., as an opaque process).

Particular embodiments may provide that the machine-readable code is any one of: a QR code, a dot pattern or a watermark.

Thus, known machine readable codes may be employed to link the medical image and the metadata in a cost-effective manner, not requiring any additional technologies and/or specific understanding by a user.

The machine-readable code in some embodiments may be embedded in one of: a part of the medical image that does not contain any medical information, a randomized part of the medical image, or an extended part of the medical image.

As a result, the machine-readable code may not obscure a view of the medical image for the remote observer. This is because the machine-readable code may be provided such that the user cannot see it, or so that it is in a different position to a structure/anatomy of the subject.

In some embodiments, the first communication channel may be adapted for real-time data transfer, and the second communication may be adapted for reliable data transfer.

Ensuring that the first communication channel is for real-time data transfer means that the remote observer may continually see an (up to date) medical image representing a result of a scan that is being conducted. As such, a latency is reduced, as the first communication channel may (although not ideally) sacrifice some quality (i.e., resolution reduction or frame rate reduction) in order to ensure prompt delivery of the medical image. This may be particularly beneficial for unreliable/inconsistent network connections.

At the same time, the metadata may be sent over a second communication channel that is adapted for reliable (i.e., high quality) data transfer. Indeed, it has been realized that it may not be necessary to transmit the metadata immediately or guarantee real-time delivery, as the metadata is likely to change at a much slower rate than the medical image.

Put another way, the medical image is likely to change drastically frame-by-frame, and therefore continual transmission of new medical images is important to facilitate real time examination. However, the metadata is likely to change much less frequently, and therefore the quality of the transmitted data is more important than the frequency of transmission. Therefore, within a certain range of subsequent image frames, the metadata associated with a first image frame within this range can also be linked to other image frames within this range. This is especially useful when (regular) image frame drops are occurring due to limited network bandwidth or other network conditions.

In specific cases, the first communication channel may be a WebRTC (Web Real-Time Communication) video channel, and the second channel may be a WebRTC data channel.

Two existing communication schemes exist in the form of web real time communication channels. WebRTC video channels are appropriate for fast, real-time communication of images, while WebRTC data channels are appropriate for reliable communication of data.

In some embodiments, the method may further comprise generating a unique code; and adding the unique code to the metadata. In this case, the identifier is based on part of the metadata corresponding to the unique code.

In other words, a unique code/identifier is generated for each medical image, metadata pair (e.g., based on a time stamp or acquisition sequence number). This unique code/identifier is then added/appended to the metadata. The identifier encoded into the medical image can thus be based on the part of the metadata that corresponds to this unique code/identifier, such that they may be matched at a receiver.

Accordingly, the metadata and medical image can be linked based on a generated unique code. The generated unique code, encoded in the medical image and present in the metadata, may also provide useful information - such as corresponding to a sequence number of the medical image or a time of acquisition, thus enabling sorting of medical images and metadata at the receiver.

In some embodiments, obtaining the medical image and the metadata may comprise fetching the medical image and the metadata from an acquisition application configured to acquire the medical image from an imaging device in real-time.

As a result, the proposed method may be used alongside existing medical image acquisition applications. This may increase a compatibility of the proposed method with existing workflows and systems.

In further embodiments, obtaining the medical image and the metadata may comprise obtaining a digital navigation link (DNL) data object comprising the medical image and the metadata, and extracting the medical image and the metadata from the DNL data object.

DNL data objects provide a simple way of encapsulating and communicating real-time medical image data (e.g., ultrasound image data).

In some embodiments, the method may further comprise serializing the metadata into a byte or string format adapted to be transferable over the second communication channel.

Thus, the metadata may be efficiently transferred over the second communication channel.

Also, the metadata may comprise an acquisition mode of the medical image. In some cases, the acquisition mode may include at least one of an image gain, an image depth mode, an image color map, an image rotation, an image translation, and an image coordinate system.

The above information may be particularly useful for a remote observer to fully understand the medical image(s) being received, as well as for real-time or future auditing of the scanning session.

In some embodiments, the medical image may be an ultrasound image. In this case, the ultrasound image may optionally comprise at least one of: a B-mode image component, a Color Flow Doppler image component, and a Pulsed Wave Doppler image component.

A duplex ultrasound scanning session producing two live streams of medical images may particularly benefit from the proposed methods. In this case, different image components may be produced, each of which may be transferred over a different communication channel.

According to further examples in accordance with an aspect of the invention there is provided a method of receiving medical imaging data for remote real-time examination, comprising: receiving an encoded medical image by a first communication channel, the encoded medical image comprising an identifier encoded in a medical image; receiving metadata by a second communication channel different from the first communication channel, wherein the metadata comprises information describing one or more parameters of the medical image, and wherein the identifier is based on at least part of the metadata; extracting the identifier from the encoded medical image; and matching the medical image and the metadata based on the identifier.

According to alternative examples in accordance with an aspect of the invention there is provided a method of communicating medical imaging data for remote real-time examination, comprising transmitting an encoded medical image and metadata according to any of the above transmitting methods, and receiving the encoded medical image and metadata according to the above receiving method.

According to other examples in accordance with an aspect of the invention, there is provided a computer program comprising computer program code adapted, when said computer program is run on a computer, to implement any of the above methods of transmitting, receiving and/or communicating medical imaging data for remote real-time examination.

According to additional examples in accordance with an aspect of the invention, there is provided a system for transmission of medical imaging data for remote real-time examination, the system being configured to carry out any of the above transmitting methods. Such a system may comprise: an interface module configured to obtain a medical image and metadata comprising information describing one or more parameters of the medical image; a processing module configured to encode the medical image with an identifier, the identifier based on at least part of the metadata; a first communication channel module configured to transmit the encoded medical image to the receiver by a first communication channel; and a second communication channel module configured to transmit the metadata to a receiver by a second communication different from the first communication channel.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 presents a flow diagram of a method of transmitting medical imaging data for remote real-time examination according to an embodiment of the invention;
Fig. 2 presents a flow diagram of a method of receiving medical imaging data for remote real-time examination according to another embodiment of the invention;
Fig. 3 presents a flow diagram of a method of communicating medical imaging data for remote real-time examination according to a further embodiment of the invention;
Fig. 4 is a simplified block diagram of a system for communicating medical imaging data for remote real-time examination according to an additional embodiment; and
Fig. 5 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings.

It should also be understood that the Figures are merely schematic and are not drawn to scale. It should also be understood that the same reference numerals are used throughout the Figures to indicate the same or similar parts.

The invention proposes concepts for communicating medical imaging data to a remote observer so that they may partake in a real-time examination of a subject. In particular, the use of an identifier encoded in a medical image facilitates the transmission of the medical image and associated metadata along different communication channels. Indeed, by the use of the identifier, the metadata and the medical image may be matched at a receiver. The use of different, separate communication channels means that said communication channels may be individually formatted/adapted/configured to suit the different requirements of transmission for the medical image and metadata.

By way of explanation, establishing a connection between a scanner/machine and a remote observer is often cumbersome. This may be particularly the case if the scanner and remote observer are not on the same subnet.

Digital navigation link (DNL) data format and communication protocols may be used to stream images/frames as structured data objects containing medical images (i.e., ultrasound images, magnetic resonance images, etc.) and metadata related to the medical images in each DNL data packet.

Moreover, real-time transfer of medical imaging data (e.g., DNL data objects) cannot be guaranteed to be achieved in real-time using existing techniques (i.e., there may be significant latency). This problem is compounded when combining the transfer of medical imaging data with two-way audio/video communication (i.e., between a person conducting the scan, and a remote observer), as is often needed for remote guidance of an examination. In this case, the bandwidth required is increased significantly, with an associated rise in latency.

While many services offer data channels for reliable data transfer, there are no real-time delivery guarantees. It is possible to transfer medical imaging data over existing data channels in real-time. However, when operating under imperfect network conditions, frequent multi-second time-outs may occur. This renders existing solutions unsuitable for guaranteed real-time medical imaging data (e.g., a DNL data object) transmission.

Some proposed embodiments may aim to overcome the above-mentioned problems by use of separate communication channels to assure real-time delivery of medical images and reliable transmission of associated metadata.

A first channel (e.g., a WebRTC video channel) may be used to send a particular medical image component. In the case that the medical imaging is spectral Doppler ultrasonography, there are three image components, i.e., B-mode, Doppler mode, and spectral waveform, requiring three of such channels that operate in real-time. In other words, a first channel may transmit a medical image component quickly, potentially at the expense of some loss of frames or resolution (although ideally lossless). A second channel (e.g., a WebRTC data channel) may be used to send the associated metadata. The second channel may operate at a low bitrate and may not necessarily be in real-time. The transfer of the medical image and associated metadata may be performed in parallel (i.e., at approximately the same time) through the two types of channels.

Accordingly, by utilising two different communication channels, the transfer of the different medical image data components may be adapted based on individual needs. In an embodiment, the medical image is transferred quickly so as to ensure real-time transfer, while the metadata is transferred in a reliable manner so as to ensure accuracy. Indeed, it has been realized that transfer of the metadata in real-time may not be necessary as the metadata may not change quickly or unpredictably between frames in the same way that the medical image data may change between frames.

Furthermore, linking of metadata to a particular medical image is possible at a receiver due to an identifier encoded/inserted/added to the medical image. The identifier is based on at least part of the metadata, possibly added to the original image metadata at the sender, such that the metadata linked to the medical image may be readily identified. In some embodiments, it may be advantageous that the medical image is encoded invisibly (i.e., not viewable by a human observer) such that the observer is not distracted/annoyed/mislead by the identifier. Alternatively, the identifier may be inserted in a part of the medical image that does not contain any image information.

In this way, metadata associated with each medical image/frame within a stream of medical images can be correlated correctly at a receiving endpoint. This may be used by the receiver or an artificial intelligence agent in the cloud to enable medical image processing and image interpretation, so as to provide fast and accurate medical diagnosis feedback at the point of care (i.e., a source of the medical imaging data).

It will be appreciated by the skilled person that embodiments of the invention may be applied to tele-guided diagnosis and intervention. For example, a relatively untrained user may be guided by a remote observer via tele-conferencing technology, while receiving medical imaging data according to an embodiment of the invention. This may save time of the skilled observer, and stress of the subject of the scan.

The additional transmission of metadata (and linking with medical images via an identifier) may be very beneficial for diagnosing and understanding of the medical images by the observer. For example, the observer may choose to subsequently input the medical image(s) and associated metadata to artificial intelligent agents that can be connected to an examination session at any time, e.g., during the same WebRTC call. Such artificial intelligence agents may require such metadata to accurately assess the situation, and thus provide accurate conclusions.

Some embodiments may support the examination itself (such as probe movements, tilts, rotations), and may provide image enhancements such as segmentations and annotations. This may aid in establishing a medical diagnosis to the user performing the examination in real-time. In such cases, a remote human observer or a remote AI agent would provide an annotated video stream back to the operator of the probe that shows probe manipulation instructions, and/or could provide a data stream that either contains manipulation instructions or is directly fed into the probe for remote control of acquisition settings.

Other embodiments also may facilitate detailed audit trails of medical examinations (stored elsewhere) and improve the guidance in an educational setting.

The invention may be applied to many imaging modalities, which may generate multiple image streams at the same time. Indeed, in these cases remote observation may be beneficial, but may be impractical due to the high level of latency experienced due to the size of such datasets and associated bandwidth requirements. Examples of these are ultrasound imaging, c-arm fluoroscopy, and multi-plane image modalities including 3D ultrasound, and multi-plane X-Ray.

In some specific exemplary embodiments of the invention, the following main features may be employed on the side of the sender in order to enable transmission of the medical imaging data:
(i) A medical imaging data acquisition module. This may be in the form of an application that may control a scanning device (e.g., an ultrasound probe) to capture and transfer medical images and metadata in real-time.
(ii) An interface module. This may fetch data from a running the medical imaging data acquisition module. Further, the interface may encode said data into DNL data objects that can be retrieved by processing and visualization applications.
(iii) A processing module. This may retrieve the medical imaging data (in the form of a DNL data object) from the interface module, and:
   (a) Extract medical images and metadata from each DNL data object;
   (b) Serialise the metadata into a network-transferable byte or string format;
   (c) Extracts a frame identifier (e.g., a frame sequence number) and encodes each medical image. For example, the medical image may be encoded with a watermark that represents the frame sequence number;
   (d) Provides the encoded images as new frames into video source modules of a video communication channel controller (e.g., a WebRTC video channel module); and
   (e) Provides the serialized metadata to a data communication channel controller (e.g. a WebRTC data channel module).
(iv) A data channel client module that sends the metadata packet to a designated destination.
(v) A video channel client module that receives the updated frames and encodes these into video streams and sends the video streams to a destination (server or peer) over a video channel.

To clarify, the processing module may generate a pattern of visible dots of a small total size (e.g., 40 x 40 pixels, containing 16 dots of 8 x 8 pixels with high luminance value) based on the frame sequence number. The pattern may be extended with a fixed (or slowly varying) unique set of dots to enable confirmation of the presence of a dot pattern as well as its location/boundary. The full pattern may then be inserted in one or more of: a fixed area that contains no information, a different location for each frame, or an area that is an extension of the original image frame (effectively increasing the image size).

In other specific exemplary embodiments of the invention, the following main features may be employed on the side of the receiver in order to capture the sent medical imaging data:
(i) A data communication channel module that upon reception of a metadata packet, checks whether the received metadata packet is a metadata packet, and if true, notifies a metadata buffer module and stores the packet in this buffer.
(ii) A metadata buffer module that, upon receiving a notification and a new metadata packet extracts a frame identifier and stores the full metadata packet in an internal array that is indexable by the extracted frame identifier.
(iii) A video communication channel module that receives an encoded video stream (i.e., a sequence of encoded medical images) and decodes each medical image. In the case that there are multiple video streams (i.e., multiple types of medical images), multiple video communication channel modules may each handle a different video stream.
(iv) A view sink module. The view sink module (or multiple in the case of multiple different video streams), upon reception of a new medical image frame:
   (a) Checks the medical image for the presence of an anticipated dot pattern at an anticipated location. If no compatible dot pattern is detected at the anticipated location, the medical image is rendered and displayed without any change. If a valid dot pattern is detected the pattern is decoded, the resultant identifier value is stored in an ID buffer and the medical image is modified.
      The modification may be one of: the dot pattern is masked to make it invisible, the medical image is enhanced with an alphanumeric string that represents the (decoded) dot pattern and is placed at the location of the dot pattern to overlay the dot pattern, and/or the medical image is reduced in size, where the hidden area is the extension of the original image where the dot pattern was placed at the sender. Alternatively, no change may be made to the medical image (i.e., the dot pattern remains a part of the full medical image).
(v) A reformatter module, which upon receiving a notification (containing the frame identifier) that there is a newly received medical image frame:
   (a) Fetches the most recent IDs from the ID buffer;
   (b) Matches (within a given range) the frame identifier with available metadata (from the metadata buffer) and available medical image frames associated with said frame identifier;
   (c) Reconstructs the original data (i.e., a DNL data packet) for storage (e.g., to be used by advanced (AI) image/data analysis tools);
   (d) Converts the medical image and metadata into a DICOM (Digital Imaging and COmmunications in Medicine) RTV (RealTime Video) stream for storage in a DICOM store; and
   (e) Renders a selected part of the metadata in a readable format and displays this in an information window or on top of one or more of displayed incoming medical image streams at a particular location. This may depend on user selected settings regarding what metadata needs to be displayed.

Of course, the above-described embodiments contain a specific example of how to implement the invention, but the skilled person would appreciate that not all indices are required to realise the invention.

To summarise, the main features that may be encapsulated by embodiments of the invention are:
(i) Reliable and real-time transmission of medical images with associated metadata;
(ii) Reliable and real-time DNL transmission;
(iii) Seamless connection setup due to the use of standard WebRTC services;
(iv) Use of standard clients, such as web browsers, as (receiving) endpoints;
(v) No frame accurate sink between metadata and image display, but visual check is possible by seeing probe and/or acquisition controls on screen, or doing a remote-control action of the acquisition that is visualized in the DNL metadata (feedback loop);
(vi) Provision of frame accurate re-linking of medical images and metadata;
(v) Enabling advanced AI applications/services for analysing the medical images at a receiver;
(vi) Enabling extensive auditing and monitoring of examinations by a remote observer for legal and educational purposes;
(vii) Providing a practical implementation for DICOM RTV transmission;
(viii) Transmission of multiple medical image streams in parallel over different video communication channels. In some examples, this may cater for different types of ultrasound, including 3D ultrasound and Bi-Planes; and
(ix) Extension with multiple bi-directional video communication channels possible using standard WebRTC solutions to provide additional audio and video guidance and inspection options.

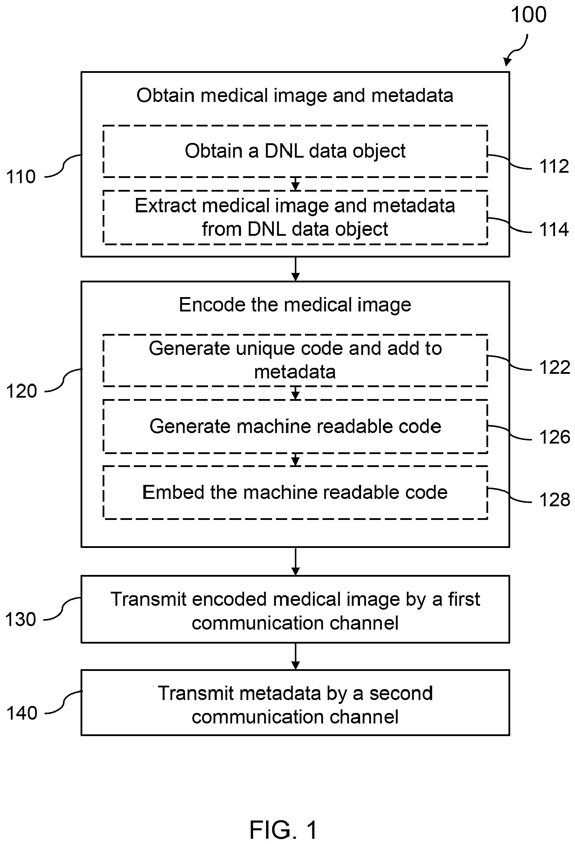

Moving on to Fig. 1, there is shown a flow diagram of a method for transmitting medical imaging data for remote real-time examination. Optional sub-steps are shown in dotted boxes.

The method is suitable for sending/transmitting medical imaging data to a receiver in such a way that a person remote (i.e., physically removed) from the examination/scanning session may be able to partake in the examination in real-time. For example, this method may be useful for a person acting in a supervisory capacity who cannot be physically present at the examination, or may be useful for teaching purposes. Of course, these circumstances benefit from being able to partake in real-time (i.e., as the examination is being conducted), as many insights, advice and guidance may be lost when reviewing the examination after the fact.

At step 110, a medical image and metadata are obtained. The metadata comprises information describing one or more parameters (properties) of the medical image.

The medical image may be a medical image of a subject, and more specifically a medical image of an anatomy/pathology/structure of a subject. For example, the medical image may be an ultrasound image, a computed tomography image, a magnetic resonance image, a position emission tomography image, etc. Indeed, it should be appreciated that the medical image may be any image that is useful in a medical context (i.e., for diagnosis/prognosis/treatment of a subject).

In some specific implementations/embodiments, the medical image is an ultrasound image. In this case, the medical image may comprise one of a B-mode component, Doppler mode component, and spectral waveform component. In some circumstances (which will be described in more detail below) a plurality of different images and image streams may be provided. Put another way, many different image components may be acquired during a single ultrasound examination session, which may all be handled and sent through different communication channels.

By way of example, the metadata describe characteristics/properties of the medical image and a context of its acquisition. Specifically, the metadata may comprise an acquisition mode of the medical image. The acquisition mode may include at least one of an image gain, an image depth mode, an image color map, and in case of 3D ultrasound, an image rotation, an image translation, and an image coordinate system. In this way, the metadata may provide a context by which a remote observer may understand the examination, or may provide additional data useful for advanced analysis techniques/algorithms.

The obtaining of the medical image and the metadata may comprise fetching the medical image and the metadata from an acquisition application configured to acquire medical images from an imaging device in real-time. For example, such acquisition applications may already exist, and therefore may be a ready source of a live stream of medical image data.

At (optional) sub-step 112, a digital navigation link (DNL) data object may be obtained. The DNL data object comprises the medical image (or multiple medical images) and the associated metadata. In this case, at (optional) sub-step 114, the medical image and the metadata are extracted from the DNL data object. In this way, the medical image and (associated/corresponding) metadata may be obtained.

At step 120, the medical image is encoded with an identifier. The identifier is based on at least part of the metadata. In this way, the medical image and the identifier are linked in the form of an encoded medical image.

In some embodiments, the identifier may be a frame sequence number (either unique, or following a predictable repeating pattern) being part of the metadata. Of course, any identifier which relates to the metadata (i.e., be based on at least part of the metadata) may be used, that may subsequently be compared to the metadata at the receiver to match the metadata with the identifier.

In further embodiments, (optional) sub-step 122 is provided. At sub-step 122, a unique code is generated. The unique code may be based on a sequence number of the acquired medical imaging data, or a time that the medical imaging data was obtained. The unique code is then added/appended to the metadata. This results in the generation of extended metadata.

When the extended metadata (including the generated unique code) is generated, the identifier encoded within the medical image is based on the unique code. In other words, the medical image is encoded with an identifier that is based on part of the metadata that corresponds to the unique code (i.e., the code added to the metadata). In this way, the medical image and metadata may be uniquely linked.

In other words, the sender may first extend the metadata with a unique code. This unique code is generated to ensure that it is unique, and can then form a unique basis for linking of the metadata and medical image. Further, the unique code may also have specific features (e.g., be based on a sequence number, and/or a time stamp) to enable approximate matching of metadata and medical images. For example, a receiver may match unique identifiers within the medical image and metadata in a certain range (metadata may be matched to previous/next medical images). Indeed, regular drops of medical images may occur in a video channel to assure real-time, low jitter and latency, while metadata is typically not dropped). Thus, by intentionally generating a unique code having specific features, inserting this unique code in the metadata, and basing the identifier encoded within the medical image on the part of the metadata corresponding to the unique code, the above advantages may be enabled.

The medical image being encoded/embedded/amalgamated with the identifier means that the medical image and the identifier are somehow linked such that they are both sent together along a first communication channel (as described below).

Furthermore, at (optional) sub-step 126 a machine-readable code is generated based on the identifier. The code is configured such that a machine may be able to read/interpret the code from a medical image in order to extract the identifier.

The machine-readable code may be generated using any known technique. For example, the machine-readable code may be one of a QR code, a dot pattern, or a watermark. As outlined previously, the machine-readable code may have one part that is based on at least a part of the metadata (i.e., the identifier), and may have another part to designate a size, boundary and presence of the machine-readable code for automatic reading by a machine at a receiver.

At (optional) sub-step 128, the machine-readable code is embedded within (i.e., placed within) the medical image.

In some embodiments, the machine-readable code is embedded in one of a part of the medical image that does not contain any medical information (i.e., surrounding the actual image of a subject, such as a dark patch), a randomized part of the medical image (i.e., changing frame-by-frame/image-by-image such that it may not be visible at a high frame rate), or an extended part of the medical image. Thus, in some cases the machine-readable code may be invisible to the viewer at the receiver (or at least does not obscure a view of the medically relevant information), even if the machine-readable code is not removed.

In other words, the medical image is modified such that it contains a machine-readable code that links the medical image to metadata. The machine-readable code may be obscured using a number of techniques, such that it is not observable by a person who is remotely observing the examination.

At step 130, the encoded medical image is transmitted to a receiver or multiple receivers, by a first communication channel. The first communication channel may be any medium by which data (in the form of a medical image) may be transmitted from a first location (the sender) to a second location (the receiver). For example, the first communication channel may be wired or wireless.

At step 140, the metadata is transmitted to the receiver by a second communication channel different from the first communication channel. The second communication channel may be any medium by which data (in the form of metadata) may be transmitted from a first location (the sender) to a second location (the receiver). For example, the second communication channel may be wired or wireless.

As an aside, step 130 and step 140 may be performed simultaneously, or step 140 may be performed at a different (i.e., later) time than step 130.

Essentially, the encoded medical image and the metadata are transmitted along different communication channels. Different communication channels may include the same physical (wired/wireless) connection, but will have separately allocated resources. Indeed, different communication channels simply means that the encoded medical image and the metadata are transmitted according to at least one of different protocols, at different times, over different networks/systems, etc. Thus, different requirements for transmission may be met.

Indeed, in exemplary embodiments, the first communication channel is adapted for real-time data transfer, and the second communication is adapted for reliable data transfer.

This means that the first communication channel may be configured/adapted such that the encoded medical images are transmitted as promptly as possible (i.e., with minimal delay/latency), potentially at the expense of a quality of the transmission. This may be achieved by allocating a larger bandwidth relative to the second communication channel, and generally prioritising resources for the first communication channel. In order to achieve real-time communication in potentially unreliable network conditions, the first communication channel may sacrifice quality (i.e., be undesirably lossy), in order to ensure that a remote observer sees the examination as it occurs.

Also, this means that the second communication may be configured such that the metadata is reliably transmitted. This means that the metadata may not be transmitted in real-time, but will be accurate.

Specifically, the first communication channel may be a WebRTC video channel, and the second channel may be a WebRTC data channel. These known channel formats are configured for real-time transmission and reliable transmission, respectively.

It should also be noted that, in some embodiments, the method may provide that prior to transmission, the metadata is serialized into a byte or string format (e.g., JSON or XML) adapted to be transferable over the second communication channel.

Overall, the method described in relation to Fig. 1 may deliver the medical image in real-time (i.e., with minimal delay/latency), while delivering the metadata reliably (i.e., with no data loss). This will depend upon the requirements of the user and the different configurations of the first and second communication channels. Of course, the skilled person implementing such a method may choose alternative requirements for the first and second communication channels, depending on desired characteristics of transmission for the medical image and the metadata.

Furthermore, it should be appreciated that the method described in reference to Fig. 1 may be continually repeated for a series/stream of medical images (i.e., a video of the examination). At least some of the medical images within the stream of medical images are encoded with the identifier to link the stream (or individual medical images) to metadata.

Moreover, in some embodiments, multiple medical images each corresponding to a different view of the examination may be obtained at once. In this case, each medical image may be transmitted via different (sub) types of first communication channels. Effectively, this provides different medial image streams. In particular, this may apply for medical image acquisitions where various different views are obtained simultaneously. For example, the medical image may be an ultrasound image, which comprises various images, such as a B-Mode image, a Pulsed Wave Doppler image, or a set of Bi-Plane images, and an X-Ray or MR (magnetic resonance) image obtained at the same time and the same area of interest.

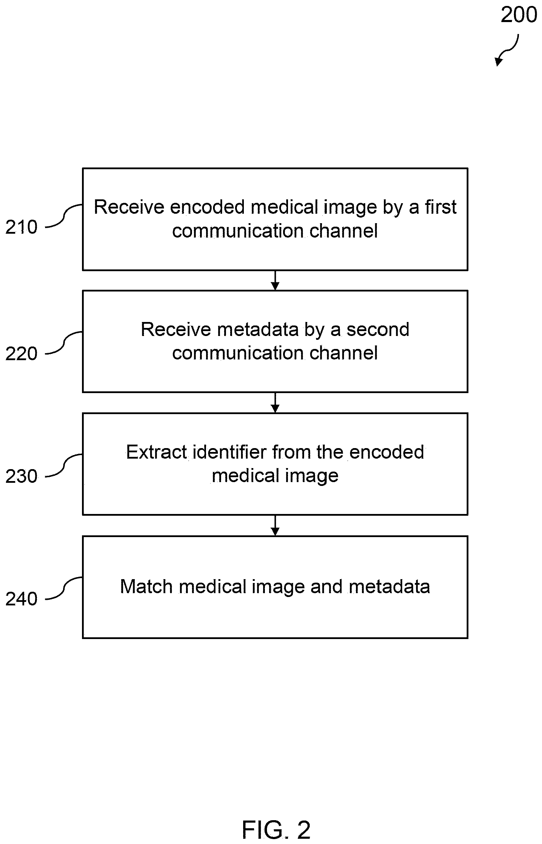

Fig. 2 presents a flow diagram of a method of receiving medical imaging data for remote real-time examination. This method may be performed at a receiver responsive to a sender transmitting the medical imaging data according to the method described in relation to Fig. 1.

At step 210, an encoded medical image is received by/via a first communication channel. The encoded medical image comprises an identifier encoded in a medical image. The identifier is based on at least part of the metadata (e.g., a frame sequence number that may have been added to the metadata at the sender before transmission) and may be in the form of a machine-readable code embedded within the medial image as described above.

At step 220, metadata is received by a second communication channel different from the first communication channel. The metadata comprises information describing one or more parameters (i.e., properties of acquisition, characteristics, etc.) of the medical image.

The first and second communication channels may be configured differently as described above, in order for the receiver to acquire the encoded medical image and metadata appropriately. For example, the first communication channel provides the medical image to the receiver in real-time (i.e., with minimal delay and latency from the examination session), while the second communication channel provides the metadata reliably (i.e., with minimal loss).

At step 230, the identifier is extracted from the encoded medical image. This may be achieved by any known technique for extracting data from a medical image. For example, known image analysis techniques may be used, which would be appreciated by the skilled person.

In one specific example, when the identifier is embedded in the medical image in the form of a machine-readable code, an optical technique may be used to identify the presence, and determine the identifier.

At step 240, the medical image and the metadata are matched based on the identifier. Indeed, because the identifier is based on at least part of the metadata, the matching may be relatively straightforward. For example, this may be achieved using known string-matching techniques.

Additionally, it may be the case that the identifier obtained from the metadata and the identifier extracted from the medical image are matched based on an interval of identifiers. For example, in a sequence of acquired medical images, not all medical images may be transmitted by the sender, and/or some may be dropped by the communication channel/network in order to preserve low-latency real-time behaviour for image transmission. However, the metadata is transmitted reliably, and hence will not be dropped by the network. As a result, an exact match of metadata to a medical image is not always possible.

Therefore, a range of identifiers could be used as a matching criteria. This may be implemented simply at the receiver if identifiers denote frame sequence numbers or time stamps. In this case, an identifier of a medical image can be matched with a nearby identifier obtained from a received metadata packet.

It should be appreciated that the method of Fig. 2 may be continually repeated for a plurality (a stream/series) of medical images. The resultant medical images (and optionally the metadata) may be presented to a remote observer. Thus, a video of the examination may be shown to the observer.

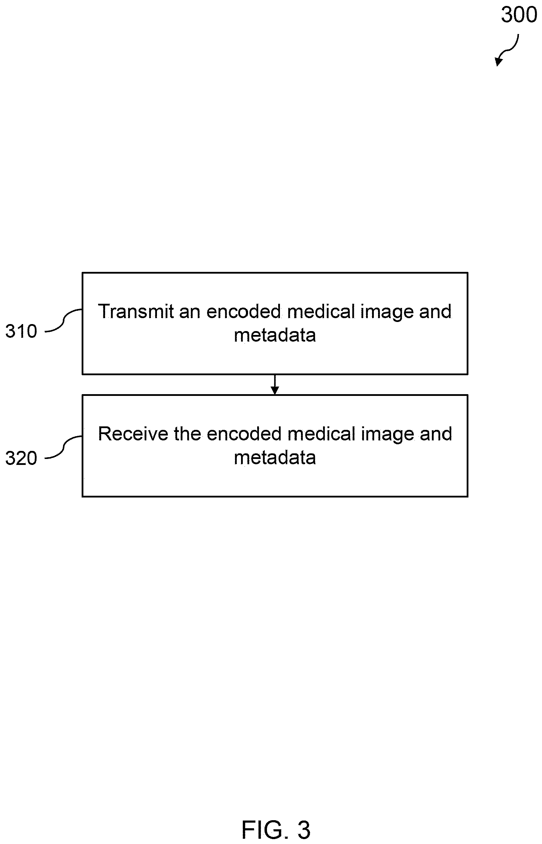

Fig. 3 presents a flow diagram of a method of communicating medical imaging data for remote real-time examination. In other words, depicted is a method whereby medical imaging data can be transferred from a sender (a user conducting a medical examination of a subject) to a receiver (a user remotely observing the examination).

Firstly, at step 310, an encoded medical image and metadata is transmitted (by a sender) according to a method described in relation to Fig. 1. Then, the encoded medical image and metadata is received (at a receiver) according to a method described in relation to Fig. 2. Thus, continual separate transmission of medical images and associated metadata may be achieved, with the metadata and medical image matched at the receiver.

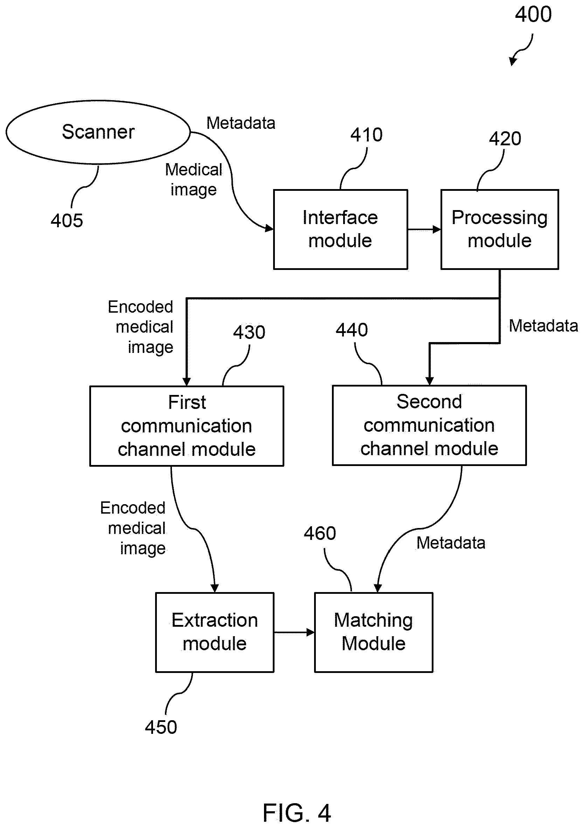

Fig. 4 contains a simplified block diagram of a system 400 for communicating medical imaging data for remote real-time examination. Specifically, there is provided a sender side including an interface module 410, a processing module 420, a first communication channel module 430, and a second communication channel module 440. There is also provided a receiver side, including an extraction module 450 and a matching module 460. Any of these modules may comprise dedicated hardware and/or a processor configured to implement software configured to implement one or more of the above method steps. Two or more of these modules may be integrated to form one combined module.

The interface module 410 is configured to obtain a medical image and metadata comprising information describing one or more parameters of the medical image. As shown, the medical image and metadata may be acquired directly from a scanner 405 (e.g., a magnetic resonance imaging (MRI) machine, an ultrasound scanner, a computed tomography (CT) machine). Alternatively, the interface module 410 may retrieve the medical image and metadata indirectly, such as from a memory or other storage.

Subsequently, the interface module 410 passes the obtained information to the processing module 420. The processing module 420 is configured to encode the medical image with an identifier, where the identifier is based on at least part of the metadata, or generated by the interface module 410 and added to the metadata. The encoded medical image and metadata are passed to the first communication channel module 430 and the second communication channel module 440, respectively.

As shown, the first communication channel module 430 is configured to transmit the encoded medical image to the receiver by a first communication channel. The second communication channel module 440 is configured to transmit the metadata to a receiver by a second communication different from the first communication channel.

The receiver may then receive the encoded medical image and the metadata via the first and second communication channels, respectively. The extraction module 450 of the receiver is configured to extract the identifier from the encoded medical image. The matching module 460 of the receiver is configured to match the medical image and the metadata based on the identifier.

As a result, the receiver acquires a medical image with associated metadata from the sender via two different communication channels. To reiterate, the communication channels are different, and therefore may be differently configured in order to meet different transmission requirements of the medical image and metadata.

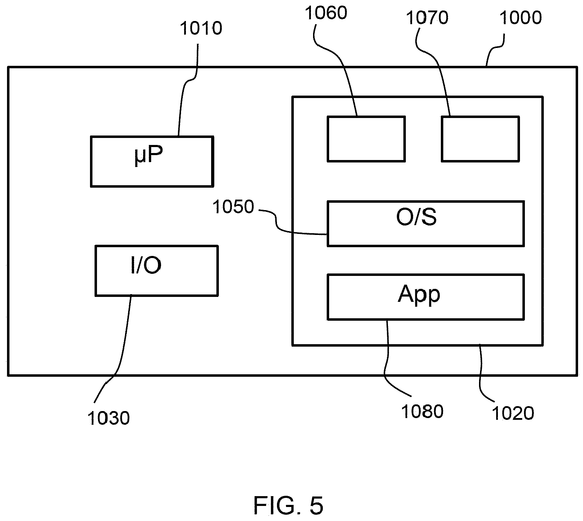

Fig. 5 illustrates an example of a computer 1000 within which one or more parts of an embodiment may be employed. Various operations discussed above may utilize the capabilities of the computer 1000. For example, one or more parts of a system for transmitting, receiving and/or communicating medical imaging data for remote real-time examination according to another embodiment of the invention may be incorporated in any element, module, application, and/or component discussed herein. In this regard, it is to be understood that system functional blocks can run on a single computer or may be distributed over several computers and locations (e.g., remotely connected via internet).

The computer 1000 includes, but is not limited to, PCs, workstations, laptops, Personal Digital Assistants, palm devices, servers, storages, and the like. Generally, in terms of hardware architecture, the computer 1000 may include one or more processors 1010, memory 1020 and one or more I/O (Input/Output) devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., read only memory (ROM), erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a Network Interface Controller or modulator/demodulator (for accessing remote devices, other files, devices, systems, or a network), a radio frequency (RF) or other transceiver, a telephonic interface, a bridge, a router, an ultrasound probe, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Figs. 1, 2 and 3, and the system(s) described in relation to Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram of Fig. 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. Measures recited in mutually different dependent claims may be advantageously combined. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method (100) of transmitting medical imaging data for remote real-time examination, the method comprising:
obtaining (110) a medical image and metadata comprising information describing one or more parameters of the medical image;
encoding (120) the medical image with an identifier, the identifier based on at least part of the metadata;
transmitting (130) the encoded medical image to a receiver by a first communication channel; and
transmitting (140) the metadata to the receiver by a second communication channel different from the first communication channel.

2. The method of claim 1, wherein encoding the medical image comprises:
generating (126) a machine-readable code based on the identifier; and
embedding (128) the machine-readable code within the medical image.

3. The method of claim 2, wherein the machine-readable code is any one of: a QR code, a dot pattern or a watermark.

4. The method of claim 2 or 3, wherein the machine-readable code is embedded in one of: a part of the medical image that does not contain any medical information, a randomized part of the medical image, or an extended part of the medical image.

5. The method of any of claims 1-4, wherein the first communication channel is adapted for real-time data transfer, and the second communication is adapted for reliable data transfer, and optionally wherein the first communication channel is a WebRTC video channel, and the second channel is a WebRTC data channel.

6. The method of any of claims 1-5, further comprising:
generating (122) a unique code; and
adding the unique code to the metadata, and wherein the identifier is based on part of the metadata corresponding to the unique code.

7. The method of any of claims 1-6, wherein obtaining the medical image and the metadata comprises fetching the medical image and the metadata from an acquisition application configured to acquire medical images from an imaging device in real-time.

8. The method of any of claims 1-7, wherein obtaining the medical image and the metadata comprises:
obtaining (112) a digital navigation link data object comprising the medical image and the metadata; and
extracting (114) the medical image and the metadata from the digital navigation link data object.

9. The method of any of claims 1-8, further comprising serializing the metadata into a byte or string format adapted to be transferable over the second communication channel.

10. The method of any of claims 1-9, wherein the metadata comprises an acquisition mode of the medical image, and optionally includes at least one of: an image gain, an image depth mode, an image color map, an image rotation, an image translation, and an image coordinate system.

11. The method of any of claims 1-10, wherein the medical image is an ultrasound image, and optionally comprises at least one of: a B-Mode image component, a Color Flow Doppler image component and a pulsed wave Doppler image component.

12. A method (200) of receiving medical imaging data for remote real-time examination, the method comprising:
receiving (210) an encoded medical image by a first communication channel, the encoded medical image comprising an identifier encoded in a medical image;
receiving (220) metadata by a second communication channel different from the first communication channel, wherein the metadata comprises information describing one or more parameters of the medical image, and wherein the identifier is based on at least part of the metadata;
extracting (230) the identifier from the encoded medical image; and
matching (240) the medical image and the metadata based on the identifier.

13. A method (300) of communicating medical imaging data for remote real-time examination, the method comprising:
transmitting (310) an encoded medical image and metadata according to a method of any of claims 1-11;
receiving (320) the encoded medical image and metadata according to the method of claim 12.

14. A computer program comprising computer program code adapted, when said computer program is run on a computer, to implement the method of any of claims 1-13.

15. A system (400) to transmit medical imaging data for remote real-time examination, the system being configured to carry out a method as claimed in any of claims 1-11.
